# EUROPÄISCHE PATENTSCHRIFT

(11) **EP 2 079 684 B1**
(45) Veröffentlichungstag und Bekanntmachung des Hinweises auf die Patenterteilung: **22.06.2016**
(21) Anmeldenummer: 07822249.4
(22) Anmeldetag: 06.11.2007
(51) Int. Cl.: C07C 263/10, C07C 265/14

(54) **VERFAHREN ZUR HERSTELLUNG VON ISOCYANATEN**
METHOD FOR PRODUCING ISOCYANATES
PROCEDE DE FABRICATION D'ISOCYANATES

(30) Priorität: 07.11.2006 EP 06123629
(43) Veröffentlichungstag der Anmeldung: 22.07.2009
(73) Patentinhaber: BASF SE, 67056 Ludwigshafen am Rhein (DE)
(72) Erfinder: DAISS, Andreas, 67146 Deidesheim (DE); WÖLFERT, Andreas, 74906 Bad Rappenau (DE); KNÖSCHE, Carsten, 67150 Niederkirchen (DE); STROEFER, Eckhard, 68163 Mannheim (DE)
(74) Vertreter: BASF IP Association
(86) Internationale Anmeldenummer: PCT/EP2007/061932
(87) Internationale Veröffentlichungsnummer: WO 2008/055899

(56) Entgegenhaltungen:
- EP-A- 1 403 248
- WO-A-2005/123665

## Beschreibung

Gegenstand der Erfindung ist ein Verfahren zur Herstellung von Isocyanaten in der Gasphase.

Isocyanate werden in großen Mengen hergestellt und dienen hauptsächlich als Ausgangsstoffe zur Herstellung von Polyurethanen. Ihre Herstellung erfolgt zumeist durch Umsetzung der entsprechenden Amine mit Phosgen.

Eine Möglichkeit der Herstellung von Isocyanaten ist die Umsetzung in der Gasphase. Die Vorteile dieser Verfahrensführung liegt in einem verringerten Phosgen-Hold-Up, in der Vermeidung schwer phosgenierbarer Zwischenprodukte und erhöhten Reaktionsausbeuten. Neben einer effektiven Vermischung der Eduktströme stellen die Realisierung eines engen Verweilzeitspektrums und die Einhaltung eines engen Verweilzeitfensters wichtige Voraussetzungen für die technische Durchführbarkeit eines derartigen Prozesses dar. Diese Anforderungen können beispielsweise durch den Einsatz von turbulent betriebenen Rohrreaktoren oder durch Strömungsrohre mit Einbauten befriedigt werden.

Aus dem Stand der Technik sind verschiedene Verfahren zur Herstellung von Isocyanaten durch Umsetzung von Aminen mit Phosgen in der Gasphase bekannt.

EP-A-593 334 beschreibt ein Verfahren zur Herstellung von aromatischen Diisocyanaten in der Gasphase, dadurch gekennzeichnet, dass die Umsetzung des Diamins mit Phosgen in einem Rohrreaktor ohne bewegte Teile und mit einer Verengung der Wände entlang der Längsachse des Rohrreaktors stattfindet. Das Verfahren ist jedoch problematisch, da die Vermischung der Eduktströme allein über eine Verengung der Rohrwand schlecht im Vergleich zur Anwendung eines richtigen Mischorganes funktioniert. Eine schlechte Vermischung führt üblicherweise zu einer unerwünscht hohen Feststoffbildung.

EP-A-699 657 beschreibt ein Verfahren zur Herstellung von aromatischen Diisocyanaten in der Gasphase, dadurch gekennzeichnet, dass die Umsetzung des zugehörigen Diamins mit dem Phosgen in einem zweizonigen Reaktor stattfindet, wobei die erste Zone, die 20 % bis 80 % des Gesamtreaktorvolumens ausmacht, ideal vermischt ist und die zweite Zone, die 80 % bis 20 % des Gesamtreaktorvolumens ausmacht, durch eine Kolbenströmung charakterisiert werden kann. Weil jedoch mindestens 20 % des Reaktionsvolumens ideal rückvermischt sind, resultiert eine ungleichmäßige Verweilzeitverteilung, die zu einer unerwünscht erhöhten Feststoffbildung führen kann.

EP-A-289 840 beschreibt die Herstellung von Diisocyanaten durch Gasphasenphosgenierung, wobei die Herstellung erfindungsgemäß in einer turbulenten Strömung bei Temperaturen zwischen 200°C und 600 °C in einem zylindrischen Raum ohne bewegte Teile stattfindet. Durch den Verzicht auf bewegte Teile wird die Gefahr eines Phosgenaustrittes reduziert. Durch die turbulente Strömung im zylindrischen Raum (Rohr) wird, wenn man von Fluidelementen in Wandnähe absieht, eine gute Strömungsgleichverteilung im Rohr und damit eine enge Verweilzeitverteilung erreicht, die, wie in EP-A-570 799 beschrieben, zu einer Verringerung der Feststoffbildung führen kann.

EP-A-570 799 betrifft ein Verfahren zur Herstellung von aromatischen Diisocyanaten in der Gasphase, dadurch gekennzeichnet, dass die Umsetzung des zugehörigen Diamins mit dem Phosgen in einem Rohrreaktor oberhalb der Siedetemperatur des Diamins innerhalb einer mittleren Kontaktzeit von 0,5 bis 5 Sekunden durchgeführt wird. Wie in der Schrift beschrieben ist, führen zu lange als auch zu kurze Reaktionszeiten zu einer unerwünschten Feststoffbildung. Es wird daher ein Verfahren offenbart, bei dem die mittlere Abweichung von der mittleren Kontaktzeit weniger als 6 % beträgt. Die Einhaltung dieser Kontaktzeit wird dadurch erreicht, dass die Reaktion in einer Rohrströmung durchgeführt wird, die entweder durch eine Reynolds-Zahl von oberhalb 4.000 oder eine Bodenstein-Zahl von oberhalb 100 charakterisiert wird.

EP-A-749 958 beschreibt ein Verfahren zur Herstellung von Triisocyanaten durch Gasphasenphosgenierung von (cyclo)aliphatischen Triaminen mit drei primären Aminogruppen, dadurch gekennzeichnet, dass man das Triamin und das Phosgen kontinuierlich in einem auf 200 °C bis 600°C erhitzten, zylindrischen Reaktionsraum mit einer Strömungsgeschwindigkeit von mindestens 3 m/s miteinander zur Reaktion bringt.

EP-A-928 785 beschreibt die Verwendung von Mikrostrukturmischern zur Phosgenierung von Aminen in der Gasphase. Nachteilig bei der Verwendung von Mikromischern ist, dass bereits kleinste Feststoffmengen, deren Entstehung bei der Synthese der Isocyanate nicht vollständig auszuschliessen ist, zum Verstopfen des Mischers führen können, was die zeitliche Verfügbarkeit der Phosgenierungsanlage herabsetzt.

In allen Fällen ist es jedoch notwendig, nach Erreichen einer optimalen Reaktionszeit die Reaktion effektiv abzubrechen, um die Bildung von Feststoffen durch Folgereaktionen des Isocyanats zu verhindern.

DE 10245704 A1 beschreibt das rasche Abkühlen eines Reaktionsgemisches, das mindestens aus einem Isocyanat, Phosgen und Chlorwasserstoff besteht, in einer Quenchzone. Die Quenchzone besteht dabei aus mindestens 2 Düsenköpfen, die ihrerseits mindestens jeweils 2 Einzeldüsen enthalten können. In der Quenchzone wird das Reaktionsgas mit den versprühten Flüssigkeitströpfchen vermischt. Durch die Verdampfung der Flüssigkeit wird die Temperatur des Gasgemisches schnell abgesenkt, so dass der Verlust des Isocyanatwertprodukts infolge hoher Temperaturen vermindert wird. Ferner wird durch die Düsenanordnung ein frühzeitiger Kontakt des heißen Reaktionsgases mit den Quenchzonenwandungen unterdrückt, so dass die Bildung von Belägen an den Oberflächen reduziert wird.

Nachteilig bei dem beschriebenen Verfahren sind die Quenchzeiten von 0,2 bis 3,0 s, die zu einem deutlichen Verlust an Isocyanat führen, den es zu vermeiden gilt.

Die internationale Patentanmeldung WO 2005/123665 beschreibt ein Verfahren, bei dem ein Reaktionsgemisch, enthaltend Isocyanat, Phosgen und Chlorwasserstoff, von der Reaktionszone in die Quenchzone durch eine Zone mit reduzierter Querschnittsfläche gelangt. Vorteilhaft bei dem beschriebenen Verfahren ist, dass auf Grund der dadurch bewirkten Geschwindigkeitserhöhung bereits eine Abkühlung des Reaktionsgemisches erreicht wird und durch die erhöhte Geschwindigkeit des die Zone mit reduzierter Querschnittsfläche verlassenden Reaktionsgemisches eine Sekundärzerstäubung der Quenchflüssigkeit erreicht wird und damit eine größere Phasengrenzfläche zwischen Reaktionsgemisch und Quenchflüssigkeit entsteht, die ihrerseits kürzere Quenchzeiten bewirkt.

Als nachteilig bei der Querschnittsverengung kann sich vor allem bei kleinen Apparateabmessungen erweisen, dass kleine Querschnittsflächen gegenüber der Ablagerung von festen Verunreinigungen oder Nebenprodukten störanfälliger sind.

Aufgabe der Erfindung war es, ein Verfahren zur Herstellung von Isocyanaten in der Gasphase zu entwickeln, das betriebsstabil ist und bei dem nach Erreichen der optimalen Verweilzeit die Reaktion innerhalb ausreichend kurzer Zeiten gestoppt wird und eine einfache Abtrennung des Isocyanats von den übrigen Bestandteilen des Reaktionsgemisches erreicht werden kann.

Die Aufgabe konnte gelöst werden durch ein Verfahren zur Herstellung von Isocyanaten aus den entsprechenden Aminen und Phosgen, in dem man die Umsetzung in der Gasphase in mindestens einer Reaktionszone durchführt und das Reaktionsgemisch zum Abbruch der Reaktion in mindestens eine Zone führt, in der man das Reaktionsgemisch zum Abbruch der Reaktion mit mindestens einer Flüssigkeit in Kontakt bringt, wobei sich zwischen der Reaktionszone und der Zone, in der man die Reaktion abbricht, ein erweiterter Bereich mit gegenüber der Reaktionszone erweitertem Querschnitt befindet, wobei das Verhältnis von Strömungsquerschnitt im erweiterten Bereich zum Strömungsquerschnitt in der Reaktionszone 1,1:1 bis 3:1 beträgt.

Als Reaktionsraum wird das Volumen beschrieben, in dem mindestens 98% des Umsatzes, d.h. der Verbrauch des eingesetzten Amins, stattfindet, bevorzugt mindestens 99,9% und speziell 99,99%. Das jenseits des Reaktionsraumes liegende Volumen stellt dann das Zwischenvolumen zwischen Reaktionsraum und Quench dar.

Die Zone, in der mindestens eine Flüssigkeit eingedüst wird, wird hier als Quenchzone bezeichnet, das Eindüsen der Flüssigkeit wird als Quenchen bezeichnet.

Die Art der Reaktionsführung und die Art der Reaktionszone spielen erfindungsgemäß keine wesentliche Rolle, solange die Phosgenierung in der Gasphase stattfindet.

Als Reaktionszone können beispielsweise Rohrreaktoren, Strömungsrohre mit oder ohne Einbauten oder Plattenreaktoren eingesetzt werden.

Die Umsetzung des Amins mit dem Phosgen in der Gasphase in der Reaktionszone kann unter den bekannten Bedingungen erfolgen.

Die Vermischung der Reaktionskomponenten Amin und Phosgen kann vor oder im Reaktor erfolgen. So ist es möglich, dem Reaktor eine Mischeinheit, beispielsweise eine Düse, vorzuschalten, wodurch bereits ein gemischter Gasstrom, enthaltend Phosgen und Amin, in den Reaktor gelangt.

In einer möglichen Ausführungsform wird zunächst der Phosgenstrom gegebenenfalls vermischt mit einem Inertmedium mittels eines Verteilelements auf die gesamte Breite des Reaktors möglichst homogen verteilt. Die Zufuhr des Aminstroms erfolgt am Anfang des Reaktors, hier ist ein Verteilerkanal mit Löchern oder Mischdüsen im Reaktionskanal eingebracht, wobei dieser Verteilerkanal bevorzugt über die gesamte Breite des Reaktors reicht. Aus den Löchern oder Mischdüsen wird das Amin, das gegebenenfalls mit einem Inertmedium vermischt ist, dem Phosgenstrom zugeführt.

Bei dem Inertmedium handelt es sich um ein Medium, das bei der Reaktionstemperatur gasförmig vorliegt und nicht mit den Edukten reagiert. Beispielsweise können Stickstoff, Edelgase wie Helium oder Argon oder Aromaten wie Chlorbenzol, Dichlorbenzol oder Xylol verwendet werden. Bevorzugt wird Stickstoff als Inertmedium verwendet.

Bevorzugt wird das erfindungsgemäße Verfahren ohne Inertmedium durchgeführt.

Für das erfindungsgemäße Verfahren können primäre Amine verwendet werden, die bevorzugt ohne Zersetzung in die Gasphase überführt werden können. Besonders geeignet sind hier Amine, insbesondere Diamine, auf Basis von aliphatischen oder cycloaliphatischen Kohlenwasserstoffen mit 1 bis 15 Kohlenstoffatomen. Beispiele hierfür sind 1,6-Diaminohexan, 1-Amino-3,3,5-trimethyl-5-aminomethylcyclohexan (IPDA), 1,3- oder 1,4-(isocyananatomethyl)cylohexan (BIC) und 4,4'-Diaminodicyclohexylmethan sowie 3 (bzw. 4), 8 (bzw. 9)-Bis(aminomethyl)-tricyclo[5.2.1.0^{2.6}]decan-Isomerengemische. Bevorzugt verwendet wird 1,6-Diaminohexan (HDA).

Für das erfindungsgemäße Verfahren können auch aromatische Amine verwendet werden, die bevorzugt ohne Zersetzung in die Gasphase überführt werden können. Beispiele für bevorzugte aromatische Amine sind Toluylendiamin (TDA), vorzugsweise 2,4- oder 2,6-Isomere oder Gemische davon, Diaminobenzol, Napthyldiamin (NDA) und 2,4'- oder 4,4'-Methylen(diphenylamin) (MDA) oder Isomerengemische davon.

Beim erfindungsgemäßen Verfahren ist es vorteilhaft, Phosgen im Überschuss zu den Aminogruppen einzusetzen. Üblicherweise liegt ein molares Verhältnis von Phosgen zu Aminogruppen von 1,1 :1 bis 20 : 1, bevorzugt von 1,2 :1 bis 5 :1 vor.

Zur Durchführung des erfindungsgemäßen Verfahrens kann es vorteilhaft sein, die Ströme der Reaktanten vor dem Vermischen vorzuwärmen, üblicherweise auf Temperaturen von 100 bis 600 °C, bevorzugt von 200 bis 500 °C. Die Umsetzung in der Reaktionszone findet üblicherweise bei einer Temperatur von 150 bis 600 °C, bevorzugt von 250 bis 500 °C statt. Das erfindungsgemäße Verfahren wird bevorzugt kontinuierlich durchgeführt.

In einer bevorzugten Ausführungsform werden die Abmessungen des Reaktors und die Strömungsgeschwindigkeiten so bemessen, dass eine turbulente Strömung, d.h. eine Strömung mit einer Reynolds-Zahl von mindestens 2300, bevorzugt mindestens 2700, vorliegt, wobei die Reynolds-Zahl mit dem hydraulischen Durchmesser des Reaktors gebildet wird. Die Reynoldszahl legt hierbei das Strömungsregime und damit die Verweilzeitverteilung im Reaktionsrohr fest (H. Schlichting: Grenzschichttheorie, Verlag G. Braun, 1982; M. Baerns: Chemische Reaktionstechnik, Georg Thieme Verlag Stuttgart, 1992).

Bevorzugt durchläuft das gasförmige Reaktionsgemisch den Reaktionsraum mit einer Strömungsgeschwindigkeit von 10 bis 300 Meter/Sekunde, bevorzugt von 25 bis 250 Meter/Sekunde, besonders bevorzugt 40 bis 230, ganz besonders bevorzugt 50 bis 200, insbesondere mehr als 150 bis 190 und speziell 160 bis 180 Meter/Sekunde.

Die mittlere Kontaktzeit des Umsetzungsgemisches in der Reaktionszone beträgt im allgemeinen zwischen 0,001 Sekunden und weniger als 5 Sekunden, bevorzugt von mehr als 0,01 Sekunden bis weniger als 3 Sekunden, besonders bevorzugt von mehr als 0,015 Sekunden bis weniger als 2 Sekunden. Im Fall der Umsetzung von (cyclo)aliphatischen Aminen kann die mittlere Kontaktzeit ganz besonders bevorzugt von 0,015 bis 1,5 Sekunden, insbesondere von 0,015 bis 0,5 Sekunden, speziell von 0,020 bis 0,1 Sekunden und oft von 0,025 bis 0,05 Sekunden betragen. Unter mittlerer Kontaktzeit wird die Zeitspanne von Beginn der Vermischung der Edukte bis zum Abbruch der Reaktion durch den Quench verstanden. In einer bevorzugten Ausführungsform ist die Strömung in der Reaktionszone im Verfahren durch eine Bodenstein-Zahl von mehr als 10, bevorzugt mehr als 100 und besonders bevorzugt von mehr als 500 charakterisiert. Die Bodensteinzahl ist dabei ein Maß für den Rückvermischungsgrad des Strömungsapparates. Mit steigender Bodensteinzahl nimmt die Rückvermischung ab (M. Baerns: Chemische Reaktionstechnik, Georg Thieme Verlag Stuttgart, 1992). Gegenstand der Erfindung ist demzufolge ein Verfahren zur Herstellung von Isocyanaten durch Umsetzung von Aminen mit Phosgen in der Gasphase in mindestens einer Reaktionszone, wobei das Reaktionsgemisch zum Abbruch der Reaktion durch mindestens eine Zone geführt wird, in der das Reaktionsgemisch mit mindestens einer Flüssigkeit in Kontakt gebracht wird, wobei man das Reaktionsgemisch zwischen der Reaktionszone und der Quenchzone durch eine Zone führt, die einen gegenüber der Reaktionszone erweiterten Querschnitt aufweist. Dabei bedeutet der Begriff "erweitert" eine Zunahme des Querschnitts in Hauptströmungsrichtung des Reaktionsgemischs. Die Erweiterung des Strömungsquerschnitts ist dabei bevorzugt so gewählt, dass das Reaktionsgas möglichst ablösungsfrei verzögert wird, so dass sich keine Totzonen ausbilden können, die anfällig für Ablagerungen sind. Wie groß der Strömungsquerschnitt oder die Erweiterung des Strömungsquerschnittes gewählt wird, orientiert sich im wesentlichen an Erfahrungen zur Verstopfungsanfälligkeit der Apparatedimensionen. So werden Verfahren mit bezogen auf die charakteristische Größe der Partikel kleinen geometrischen Abmaßen bevorzugt mit einer großen Querschnittserweiterung gestaltet.

Dabei sind stark ablagerungsbildende Isocyanate besonders Monoisocyanate und (cyclo)aliphatische Isocyanate, insbesondere 1,6-Hexamethylendiisocyanat.

Hingegen sind wenig ablagerungsbildende Isocyanate beispielsweise aromatische Isocyanate und insbesondere Toluylendiisocyanat.

Als allgemeine Regel steigt die Neigung von Isocyanaten zur Bildung von Ablagerungen mit steigender Funktionalität, steigender Reaktivität und/oder steigendem Molgewicht.

Die Machzahl der Strömung in der Zwischenzone beträgt beispielsweise 0,05 bis 0,95, bevorzugt 0,1 bis 0,9 und besonders bevorzugt 0,2 bis 0,85. Unter der Machzahl versteht man hierbei die lokale Strömungsgeschwindigkeit bezogen auf die lokale Schallgeschwindigkeit des Reaktionsgemisches. Aus der Anforderung an die Machzahl ergibt sich bei vorgegebenem Massenstrom direkt die Größe des Querschnitts im Zwischenvolumen zwischen Reaktionsraum und Quench.

Das Verhältnis von Strömungsquerschnitt im erweiterten Bereich zum Strömungsquerschnitt in der Reaktionszone beträgt 1,1:1 bis 3:1 und bevorzugt 1,2:1 bis 2:1.

Mit dem Begriff "Strömungsquerschnitt" ist damit die Querschnittsfläche gemeint, also die von den Innenmaßen des Apparats begrenzte Fläche senkrecht zur Hauptströmung des Reaktionsgemischs. Das Verhältnis der Strömungsquerschnitte wird dabei gebildet zwischen dem kleinstmöglichen Strömungsquerschnitt der Reaktionszone und dem Strömungsquerschnitt am Eingang zum Quench.

Dabei ist im Falle der Querschnittserweiterung die Form und Länge des Übergangsbereiches zwischen Reaktionszone und Eintritt in den Quenchbereich bevorzugt so zu wählen, dass möglichst keine Ablösung der Strömung auftritt. Wie der Übergang in Abhängigkeit von der Querschnittsform der Reaktionszone und des Eintrittsquerschnittes gestaltet werden muss, damit keine Ablösung auftritt, ist dem Fachmann bekannt. Im Falle beispielsweise eines geraden kreisförmigen Reaktionsrohres und kreisförmigen Eintrittsquerschnittes in die Quenchzone kann eine Ablösung durch die Wahl eines Übergangskonus mit einem Konushalbwinkels von beispielsweise kleiner 20°, bevorzugt kleiner 15°, besonders bevorzugt kleiner 10°, ganz besonders bevorzugt kleiner 7,5° und insbesondere kleiner 5° erreicht werden. Der Konushalbwinkel ist der Winkel, den die Wandung in der Erweiterung mit der Längsachse des Reaktionsrohres bildet.

Die Übergänge zwischen den Abschnitten können stufig oder abgerundet ausgeführt werden. Bevorzugt wird eine abgerundete Ausführung.

Die Länge der Zwischenzone ergibt sich trigonometrisch aus dem Konushalbwinkel und den Verhältnissen der Strömungsquerschnitte.

In der Quenchzone wird das Reaktionsgemisch, das im wesentlichen aus den Isocyanaten, Phosgen und Chlorwasserstoff besteht, intensiv mit der eingedüsten Flüssigkeit vermischt. Die Vermischung erfolgt derart, dass die Temperatur des Reaktionsgemisches ausgehend von 150 bis 600 °C, bevorzugt 250 bis 500°C in der Reaktionszone dann in der Quenchzone um 50 bis 300°C, vorzugsweise um 100 bis 250°C abgesenkt wird und das im Reaktionsgemisch enthaltene Isocyanat durch Kondensation und Löslichkeit vollständig oder teilweise in die eingedüsten Flüssigkeitströpfchen übergeht, während das Phosgen und der Chlorwasserstoff im wesentlichen vollständig in der Gasphase verbleiben. Das Reaktionsgemisch wird durch den Quench bevorzugt auf eine Temperatur von 100 bis 200 °C, bevorzugt auf 120 bis 180 °C, besonders bevorzugt 120 bis 150 °C am Ende der Quenchzone abgesenkt.

Der Anteil des im gasförmigen Reaktionsgemisch enthaltenen Isocyanats, das in der Quenchzone bei einer Temperatur von 120 bis 150 °C in die Flüssigphase übergeht, beträgt dabei vorzugsweise 20 bis 100 Gew.-%, besonders bevorzugt 40 bis 100 Gew.% und insbesondere 50 bis 100 Gew.-%, bezogen auf das im Reaktionsgemisch enthaltene Isocyanat.

Der Anteil des im gasförmigen Reaktionsgemisch enthaltenen Chlorwasserstoff, der in der Quenchzone in die Flüssigphase übergeht, beträgt dabei vorzugsweise unter 20 Gew.-%, besonders bevorzugt unter 15 Gew.-%, ganz besonders bevorzugt unter 10 Gew.-%, insbesondere unter 5 Gew.-% und speziell unter 2 Gew%.

Der Anteil des im gasförmigen Reaktionsgemisch enthaltenen Phosgen, der in der Quenchzone in die Flüssigphase übergeht, beträgt dabei vorzugsweise unter 20 Gew.%, besonders bevorzugt unter 15 Gew.-%, ganz besonders bevorzugt unter 10 Gew.% und insbesondere unter 5 Gew.-%.

Das Reaktionsgemisch durchströmt die Quenchzone vorzugsweise von oben nach unten. Unterhalb der Quenchzone ist ein Sammelbehälter angeordnet, in dem die Flüssigphase abgeschieden, gesammelt und über einen Auslass entfernt und anschließend in an sich bekannter Weise aufgearbeitet wird. Die verbleibende Gasphase wird über einen zweiten Auslass entfernt und ebenfalls in an sich bekannter Weise aufgearbeitet.

Die Flüssigkeitströpfchen im Quench werden mittels Ein- oder Zweistoffzerstäuberdüsen, vorzugsweise Einstoffzerstäuberdüsen, erzeugt und besitzen vorzugsweise einen Sauter-Durchmesser D₃₂ von 5 bis 5000 µm, besonders bevorzugt 5 bis 500 µm und insbesondere 5 bis 250 µm.

Der Sauterdurchmesser D₃₂ (Sauter mean diameter, SMD) beschreibt bis auf einen konstanten Faktor das Verhältnis von mittlerem Tropfenvolumen zu mittlerer Tropfenoberfläche (siehe dazu K. Schwister: Taschenbuch der Verfahrenstechnik, Fachbuchverlag Leipzig, Carl Hanser Verlag 2003) und ist somit die für den Quenchprozess wesentliche Kenngröße der erzeugten Tropfengrößenverteilung. Es ist der Tropfendurchmesser, bei dem das Verhältnis Volumen/Oberfläche das gleiche ist wie für die Summe aller Tropfen im betrachteten Ensemble und gibt den Feinheitsgrad der Zerstäubung hinsichtlich der Partikeloberfläche an.

Die Zerstäuberdüsen erzeugen je nach Ausführungsform einen Sprühkegelwinkel α von 10 bis 140°, bevorzugt von 10 bis 120°, besonders bevorzugt von 10° bis 100°.

Die Anzahl der Zerstäuberdüsen ist nicht begrenzt und kann beispielsweise je Einlaß für das Reaktionsgemisch 1 bis 10, bevorzugt 1 bis 6, besonders bevorzugt 1 bis 4, ganz besonders bevorzugt 1 bis 3 und insbesondere 1 bis 2 betragen.

Bevorzugt wird in die Quenchzone der Austrag aus einer Reaktionszone geleitet, es können aber auch die Austräge aus mehreren Reaktionszonen über einen oder mehrere Einlässe in eine Quenchzone geführt werden.

Es ist auch möglich, den Austrag aus einer Reaktionszone aufzuteilen und über mehrere Einlässe in eine oder mehrere Quenchzonen zu führen.

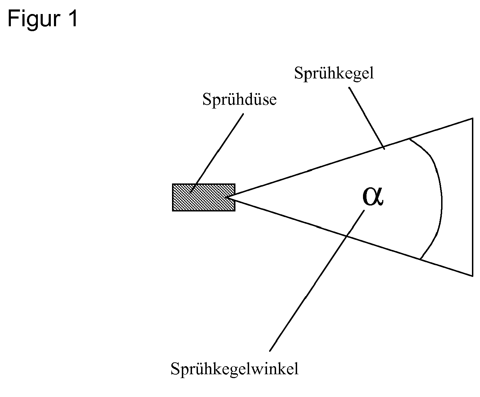

Figur 1 zeigt die Definition des Sprühkegelwinkels α: Der Sprühkegelwinkel α ist der Winkel in der Spitze des Kegels, der das Volumen einschließt, in dem sich 50 Massenprozent aller Tröpfchen bewegen.

Erfindungsgemäß befindet sich zwischen der Reaktionszone und der Quenchzone je nach Apparatedimension eine Querschnittserweiterung, durch die im Falle einer Unterschallströmung in der Reaktionszone eine geringfügige Kompression, verbunden mit einer Konzentrationsänderung, der Bestandteile des Reaktionsgases erzielt wird. Da im Vergleich zu einer Anordnung mit deutlicher Querschnittsverengung zwischen Reaktionszone und Quenchzone nur in geringerem Maße eine Sekundarzerstäubung der Quenchflüssigkeit durch den Reaktionsgemischstrom erreicht werden kann, muss dafür Sorge getragen werden, dass die in die Quenchzone eingespeisten Tröpfchen bereits einen sehr geringen Sauterdurchmesser und damit eine besonders hohe spezifische Tropfenoberfläche haben, so dass die angestrebte schnelle Temperaturabsenkung des Reaktionsgemisches erreicht werden kann.

Dadurch kann der Verlust von Isocyanatwertprodukt infolge Weiterreaktion zu Nebenprodukten minimiert werden.

Der freie Strömungsquerschnitt in der Quenchzone beträgt bezogen auf den freien Strömungsquerschnitt in der Reaktionszone 25 / 1 bis 1 / 2 bevorzugt 10 / bis 1 / 1.

Die Flüssigkeit, die über die Zerstäuberdüsen eingedüst wird, muss eine gute Löslichkeit von Isocyanaten aufweisen. Vorzugsweise werden organische Lösungsmittel eingesetzt. Insbesondere eingesetzt werden aromatische Lösungsmittel, die vorzugswesie mit Halogenatomen substituiert sein können. Beispiele für derartige Flüssigkeiten sind Toluol, Benzol, Nitrobenzol, Anisol, Chlorbenzol, Dichlorbenzol (ortho, para), Trichlorbenzol, Xylol, Hexan, Diethylisophthalat (DEIP), sowie weiterhin Tetrahydrofuran (THF), Dimethylformamid (DMF) und deren Gemische.

In einer bevorzugten Ausführungsform des erfindungsgemäßen Verfahrens handelt es sich bei der eingedüsten Flüssigkeit um ein Gemisch aus Isocyanaten, ein Gemisch aus Isocyanaten und Lösungsmittel oder um Isocyanat, wobei die jeweils verwendete Quenchflüssigkeit Anteile an Leichtsieder wie Chlorwasserstoff und Phosgen aufweisen kann. Vorzugsweise wird dabei das Isocyanat eingesetzt, das bei dem jeweiligen Verfahren hergestellt wird. Da durch die Temperatursenkung in der Quenchzone die Reaktion zum Stillstand kommt, können Folge- und Nebenreaktionen mit den eingedüsten Isocyanaten im wesentlichen ausgeschlossen werden. Der Vorteil dieser Ausführungsform liegt insbesondere darin, dass auf eine Abtrennung des Lösungsmittels verzichtet werden kann.

Die Temperatur der eingedüsten Flüssigkeit liegt vorzugsweise bei 0 bis 300°C, besonders bevorzugt bei 50 bis 250°C und insbesondere bei 70 bis 200°C, so dass bei der eingedüsten Flüssigkeitsmenge die gewünschte Abkühlung und Kondensation des Isocyanates erreicht wird.

Bevorzugt weist die eingedüste Flüssigkeit eine geringe Löslichkeit für Phosgen und/oder Chlorwasserstoff auf. Besonders bevorzugt wird die Temperatur der eingedüsten Flüssigkeit so hoch gewählt, daß sich insbesondere die gasförmigen Komponenten Phosgen und/oder Chlorwasserstoff gemäß dem Henry'schen Gesetz nur wenig in der Quenchflüssigkeit lösen.

Die Geschwindigkeit des Reaktionsgases in der Quenchzone ist vorzugsweise größer als 1 m/s, besonders bevorzugt größer als 10 m/s und insbesondere größer als 20 m/s.

Um eine schnelle Abkühlung der gasförmigen Reaktionsmischung in der Quenchzone und einen schnellen Übergang des Isocyanats in die Flüssigphase zu erreichen, müssen die Tröpfchen der eingedüsten Flüssigkeit sehr schnell über den gesamten Strömungsquerschnitt des Reaktionsgases fein verteilt werden. Die gewünschte Temperaturabsenkung und der gewünschte Übergang des Isocyanats in die Tröpfchen erfordert dabei in der Regel 10⁻⁴ Sekunden oder mehr, besonders bevorzugt mindestens 5 × 10⁻⁴ Sekunden und insbesondere mindestens 0,001 Sekunden. Die gewünschte Temperaturabsenkung und der gewünschte Übergang des Isocyanats in die Tröpfchen wird dabei vorzugsweise in bis zu 10 Sekunden, besonders bevorzugt in bis zu 1 Sekunde und insbesondere in bis zu 0,2 Sekunden durchgeführt.

Die Zeiten für den Übergang des Isocyanats in die Tröpfchen sind dabei definiert als der Zeitraum zwischen dem Eintritt des Reaktionsgases in den Quenchbereich und dem Zeitpunkt, zu dem das Reaktionsgas noch 10% von der adiabaten Endtemperatur des Gemisches aus Reaktionsgas und Tropfen abweicht. Durch die gewählten Zeiträume kann ein Verlust von Isocyanat durch Neben- bzw. Weiterreaktionen praktisch vollständig vermieden werden.

Die Geschwindigkeit, mit der die Tröpfchen aus der Düse austreten, hängt von der Art der Zerstäubung ab und beträgt in der Regel mindestens 15 m/s, bevorzugt mindestens 40 m/s und besonders bevorzugt mindestens 100 m/s. Die Obergrenze der Geschwindigkeit ist nicht kritisch. Haüfig ist eine Geschwindigkeit bis zu 350 m/s ausreichend.

Das Masseverhältnis von eingedüster Flüssigkeitsmenge zur Menge der gasförmigen Reaktionsmischung beträgt vorzugsweise 100:1 bis 1:10, besonders bevorzugt 50:1 bis 1:5 und insbesondere 10:1 bis 1:2.

Wie oben ausgeführt, wird im Anschluß an die Reaktionszone eine Quenchzone angeordnet. Die aus der Quenchzone entnommene Flüssigphase und Gasphase werden aufgearbeitet. Bei Verwendung eines Lösungsmittels als zerstäubte Flüssigkeit erfolgt eine Trennung von Isocyanat und Lösungsmittel, zumeist mittels an sich bekannter Destillation. Die Gasphase, die im wesentlichen Phosgen, Chlorwasserstoff und gegebenenfalls nicht abgetrenntes Isocyanat enthält, kann ebenfalls, vorzugsweise durch an sich bekannter Destillation oder Adsorption, in ihre Bestandteile zerlegt werden, wobei das Phosgen wieder der Reaktion zugeführt werden kann und der Chlorwasserstoff entweder für weitere chemische Reaktionen genutzt, zu Salzsäure weiterverarbeitet werden oder wieder in Chlor und Wasserstoff gespalten werden kann.

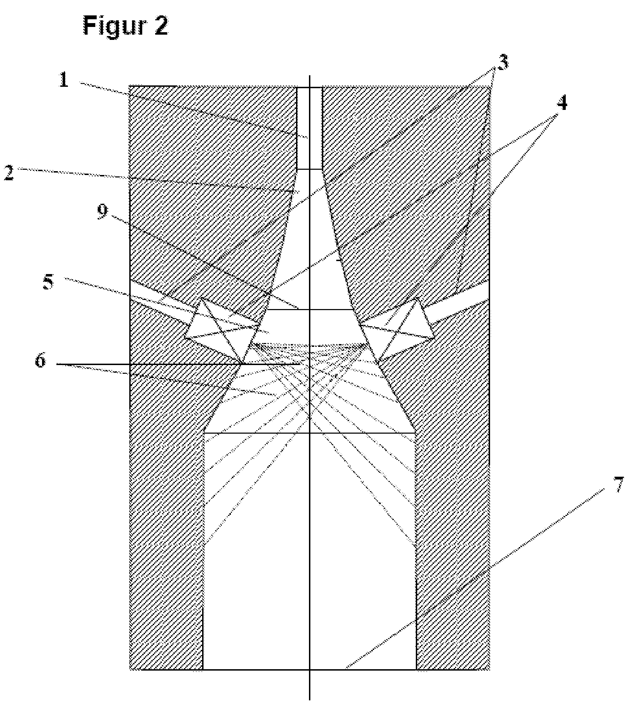

Figur 2 zeigt eine Ausführungsform des erfindungsgemäßen Verfahrens mit sich erweiternder Querschnittsfläche zwischen Reaktor und Quench.

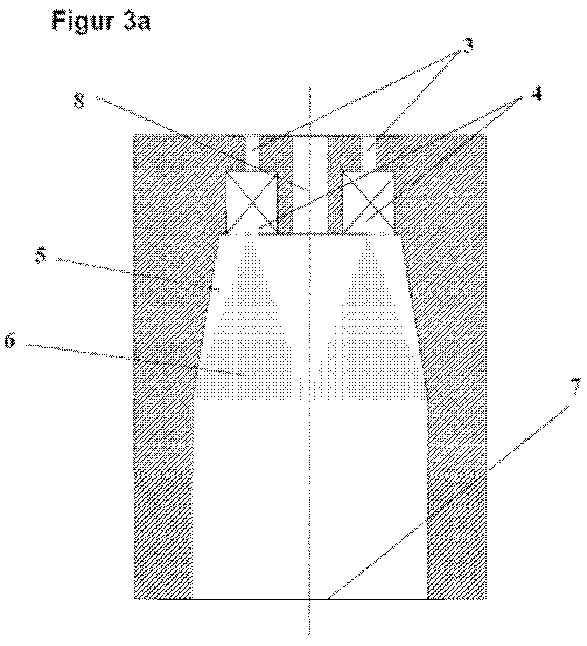

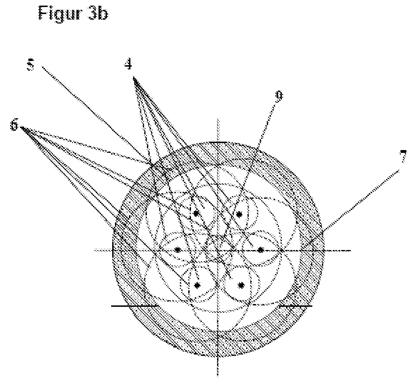

Figur 3 zeigt ein Verfahren mit gleichbleibender Querschnittsfläche zwischen Reaktor und Quench. Figur 3a: Seitenansicht, Figur 3b: Schnitt quer zur Längsachse, Blickrichtung aus Richtung der Quenchzone.

Die Erfindung soll an den nachstehenden Beispielen näher erläutert werden.

### Beispiel 1:

In einem Rohrreaktor (Durchmesser 8 mm) mit vorgeschaltendem Mischorgan wurden 67,5 kg/h Reaktionsgas erzeugt, das Toluoldiisocyanate (2,4- und 2,6-Isomerengemisch), Phosgen und Salzsäure enthielt.

Das Reaktionsgas wurde dann über eine Querschnittserweiterung mit einem Durchmesser von 10,0 mm der Quenchzone zugeführt. Der Öffnungshalbwinkel des konisch ausgeführten Erweiterungsstückes betrug dabei 5°. In der Quenchzone befanden sich zwei einzelne Einstoffdüsen mit einem Sprühkegelöffnungswinkel von 80°. Die Düsen erzeugten dabei Tropfen mit einem Sauterdurchmesser D₃₂ von ca. 100 µm. Die eingedüste Flüssigkeitsmenge betrug 100 kg/h. Die eingedüste Quenchflüssigkeit bestand aus Monochlorbenzol. Die Temperatur des Reaktionsgases beim Eintritt in die Quenchzone betrug 384°C und der Druck des Gases 10,0 bar. Die Eintrittstemperatur der Quenchflüssigkeit betrug 100°C, die Austrittsgeschwindigkeit der Flüssigkeitströpfchen aus der Sprühdüse betrug ca. 50 m/s. Die Aufenthaltszeit des Reaktionsgases in der Quenchzone betrug ca. 0,015 s. Dabei fiel die Temperatur des Quenchgases auf ca. 156°C ab. Die gewünschte Temperaturabsenkung erfolgte damit in weniger als 0,015 s. Die Toluoldiisocyanatmenge im Reaktionsgasgemisch nahm um 80 % gegenüber der Eingangskonzentration in der Quenchzone ab.

### Liste der Bezugszeichen in den Figuren:

- 1: Teil der Reaktionszone
- 2: Zone mit konischer Querschnittserweiterung
- 3: Zuführung für Quenchflüssigkeit
- 4: Zerstäubungseinrichtung
- 5: Quenchzone
- 6: Sprühkegel
- 7: Flüssigkeits- und Gasaustritt
- 8: Zone mit gleichbleibendem Querschnitt
- 9: Eintritt für Reaktionsgemisch

## Patentansprüche

1. Verfahren zur Herstellung von Isocyanaten aus den entsprechenden Aminen und Phosgen, in dem man die Umsetzung in der Gasphase in mindestens einer Reaktionszone durchführt und das Reaktionsgemisch zum Abbruch der Reaktion in mindestens eine Zone führt, in der man zum Abbruch der Reaktion mindestens eine Flüssigkeit eindüst, wobei sich zwischen der Reaktionszone und der Zone, in der man den Reaktionsabbruch herbeiführt, ein erweiterter Bereich mit erweitertem Querschnitt befindet, wobei das Verhältnis von Strömungsquerschnitt im erweiterten Bereich zum Strömungsquerschnitt in der Reaktionszone 1,1:1 bis 3:1 beträgt.

2. Verfahren nach Anspruch 1, **dadurch gekennzeichnet, dass** als Reaktionszone Rohrreaktoren, Strömungsrohre mit oder ohne Einbauten oder Plattenreaktoren eingesetzt werden.

3. Verfahren gemäß einem der vorstehenden Ansprüche, **dadurch gekennzeichnet, dass** der freie Strömungsquerschnitt in der Quenchzone bezogen auf den freien Strömungsquerschnitt in der Reaktionszone 25 / 1 bis 1 / 2 beträgt.

4. Verfahren gemäß einem der vorstehenden Ansprüche, **dadurch gekennzeichnet, dass** das Verhältnis von Strömungsquerschnitt im erweiterten Bereich zum Strömungsquerschnitt in der Reaktionszone 1,2:1 bis 2:1 beträgt.

5. Verfahren gemäß einem der vorstehenden Ansprüche, **dadurch gekennzeichnet, dass** in dem Bereich der Erweiterung die Wandung mit der Längsachse einen Konushalbwinkel von weniger als 20° einschließt.

6. Verfahren gemäß einem der vorstehenden Ansprüche, **dadurch gekennzeichnet, dass** die eingedüsten Flüssigkeitstropfen einen Sauter-Durchmesser von 5 bis 5000 µm aufweisen.

7. Verfahren gemäß einem der vorstehenden Ansprüche, **dadurch gekennzeichnet, dass** die Temperatur des Reaktionsgemisches 150 bis 600 °C beträgt.

8. Verfahren gemäß einem der vorstehenden Ansprüche, **dadurch gekennzeichnet, dass** die eingedüste Flüssigkeit ein organisches Lösungsmittel ist.

9. Verfahren gemäß einem der vorstehenden Ansprüche, **dadurch gekennzeichnet, dass** die eingedüste Flüssigkeit aromatische Lösungsmittel, die mit Halogenatomen substituiert sein können, sind.

10. Verfahren gemäß einem der Ansprüche 1 bis 7, **dadurch gekennzeichnet, dass** die eingedüste Flüssigkeit ein Isocyanat ist.

## Claims

1. A process for preparing isocyanates from the corresponding amines and phosgene, in which the reaction is carried out in the gas phase in at least one reaction zone and the reaction mixture is fed into at least one zone into which at least one liquid is sprayed to stop the reaction, wherein a widened region having a widened cross section is present between the reaction zone and the zone in which the reaction is stopped, wherein the ratio of the flow cross section in the widened region to the flow cross section in the reaction zone is from 1.1:1 to 3:1.

2. The process according to claim 1, wherein tube reactors, flow tubes with or without internals or plate reactors are used as reaction zone.

3. The process according to either of the preceding claims, wherein the ratio of the free flow cross section in the quench zone to the free flow cross section in the reaction zone is from 25/1 to 1/2.

4. The process according to any of the preceding claims, wherein the ratio of the flow cross section in the widened region to the flow cross section in the reaction zone is from 1.2:1 to 2:1.

5. The process according to any of the preceding claims, wherein, in the region of the widening, the wall makes a cone half-angle of less than 20° with the longitudinal axis.

6. The process according to any of the preceding claims, wherein the liquid droplets sprayed in have a Sauter mean diameter of from 5 to 5000 µm.

7. The process according to any of the preceding claims, wherein the temperature of the reaction mixture is from 150 to 600°C.

8. The process according to any of the preceding claims, wherein the liquid sprayed in is an organic solvent.

9. The process according to any of the preceding claims, wherein the liquid sprayed in is an aromatic solvent which may be substituted by halogen atoms.

10. The process according to any of claims 1 to 7, wherein the liquid sprayed in is an isocyanate.

## Revendications

1. Procédé de fabrication d'isocyanates à partir des amines correspondantes et de phosgène, selon lequel la réaction est réalisée dans la phase gazeuse dans au moins une zone de réaction et le mélange réactionnel est introduit pour interrompre la réaction dans au moins une zone dans laquelle au moins un liquide est injecté pour interrompre la réaction, une zone élargie présentant une section transversale élargie se trouvant entre la zone de réaction et la zone dans laquelle l'interruption de la réaction est réalisée, le rapport entre la section transversale d'écoulement dans la zone élargie et la section transversale d'écoulement dans la zone de réaction étant de 1,1:1 à 3:1.

2. Procédé selon la revendication 1, **caractérisé en ce que** des réacteurs tubulaires, des tubes d'écoulement avec ou sans composants internes ou des réacteurs à plaques sont utilisés en tant que zone de réaction.

3. Procédé selon l'une quelconque des revendications précédentes, **caractérisé en ce que** la section transversale d'écoulement dans la zone de désactivation par rapport à la section transversale d'écoulement libre dans la zone de réaction est de 25/1 à 1/2.

4. Procédé selon l'une quelconque des revendications précédentes, **caractérisé en ce que** le rapport entre la section transversale d'écoulement dans la zone élargie et la section transversale d'écoulement dans la zone de réaction est de 1,2:1 à 2:1.

5. Procédé selon l'une quelconque des revendications précédentes, **caractérisé en ce que**, dans la zone de l'élargissement, la paroi forme avec l'axe longitudinal un demi-angle de cône de moins de 20°.

6. Procédé selon l'une quelconque des revendications précédentes, **caractérisé en ce que** les gouttes de liquide injectées présentent un diamètre de Sauter de 5 à 5 000 µm.

7. Procédé selon l'une quelconque des revendications précédentes, **caractérisé en ce que** la température du mélange réactionnel est de 150 à 600 °C.

8. Procédé selon l'une quelconque des revendications précédentes, **caractérisé en ce que** le liquide injecté est un solvant organique.

9. Procédé selon l'une quelconque des revendications précédentes, **caractérisé en ce que** le liquide injecté est un solvant aromatique, qui peut être substitué avec des atomes d'halogène.

10. Procédé selon l'une quelconque des revendications 1 à 7, **caractérisé en ce que** le liquide injecté est un isocyanate.
